# EUROPEAN PATENT APPLICATION

(11) **EP 2 666 467 A1**
(43) Date of publication of application: **27.11.2013**
(21) Application number: 12737027.8
(22) Date of filing: 19.01.2012
(51) Int. Cl.: A61K 35/60, A23L 1/30, A61K 8/64, A61K 8/98, A61K 38/00, A61K 38/17, A61P 17/16, A61P 29/00, A61Q 19/08, C07K 14/46, C08B 37/00

(54) **EXTRACT OF AQUATIC ANIMAL CARTILAGE**

(30) Priority: 19.01.2011 JP 2011009284
(71) Applicant: Hirosaki University, Hirosaki-shi, Aomori 036-8560 (JP); Sunstar Inc., Takatsuki-shi Osaka 569-1195 (JP)
(72) Inventor: KATO, Yoji, Hirosaki-shi, Aomori 036-8560 (JP); KATAGATA, Yohtaro, Hirosaki-shi, Aomori 036-8560 (JP); GOTO, Masashi, Takatsuki-shi, Osaka 569-1195 (JP); YAMAMOTO, Kazushi, Takatsuki-shi, Osaka 569-1044 (JP); MAEDA, Mariko, Takatsuki-shi, Osaka 569-1195 (JP); HANADA, Yukako, Takatsuki-shi, Osaka 569-1044 (JP); TAKAI, Motoko, Takatsuki-shi, Osaka 569-1044 (JP); SUEKAWA, Yutaka, Takatsuki-shi, Osaka 569-1044 (JP)
(74) Representative: TBK
(86) International application number: PCT/JP2012/051131
(87) International publication number: WO 2012/099224

(57) **Abstract**

An object of the present invention is to obtain a useful extract from aquatic animal tissues. The present invention provides a fish cartilage water extract containing a proteoglycan having a molecular weight of not less than 2,500,000 (250 × 10⁴). The extract has a superior skin anti-aging effect (in particular, anti-inflammatory effect, skin barrier functionality improving effect, and skin water-retention ability improving effect). Moreover, the fish cartilage water extract of the present invention provides these effects not only through application to skin but also through oral administration.

## Description

### Technical Field

The present invention relates to an extract of aquatic animal cartilage. More specifically, the present invention relates to an aquatic animal cartilage extract containing a proteoglycan having a high molecular weight.

### Background Art

Proteoglycan is one of the major biological macromolecules for forming the substrate of the extracellular matrix of connective tissue, as are collagen and others. Proteoglycan was hitherto obtained by being extracted and isolated from mammal cartilage (in particular, bovine cartilage). However, ever since the occurrence of bovine spongiform encephalopathy (BSE) was reported, the use of bovine cartilage has been avoided. Thus, there has been a need for an alternative source of proteoglycan, and a production method for the alternative source. In addition, since the production cost for proteoglycan is very high due to the complexity of the existing proteoglycan extraction step and the low yield, industrial application of proteoglycan has not been fully accomplished. Thus, there has been a need for a simpler method ensuring a greater yield.

As an alternative source for proteoglycan, aquatic animal tissue is attracting attention. Therefore, there have been attempts to extract proteoglycan from cartilage of aquatic animals, such as whales and sharks. However, due to catch restrictions placed on these aquatic animals, it has been difficult to produce a large amount of proteoglycan. Moreover, the extraction and isolation of proteoglycan is complicated, and some solvents, etc., used for extraction have high toxicity.

Under such circumstances, there have been attempts to extract proteoglycan from the large amount of discarded aquatic animal tissues. For example, Patent Document 1 discloses a method for producing a composition (nasal cartilage powder) containing proteoglycan from salmon nasal cartilage by using ethanol.

### Citation List

### ■ Patent Documents

Patent Document 1: JP2009-173702A

### Summary of Invention

### ■ Technical Problem

An object of the present invention is to efficiently extract proteoglycan from aquatic animal tissues.

### ■ Solution to Problem

Surprisingly, the inventors of the present invention found a method of extracting proteoglycan from fish cartilage, which is aquatic animal cartilage, by using water. Moreover, the inventors further found that the fish cartilage water extract extracted from fish cartilage through this method contains proteoglycan of a molecular weight higher than that of the proteoglycan contained in the fish cartilage extracts obtained through hitherto known methods; further, they found that, the high molecular weight proteoglycan has a superior effect. After several attempts to further improve the extract based on these findings, the inventors completed the present invention.

Specifically, the present invention encompasses the inventions in the following items.
Item 1
   A water extract of fish cartilage comprising a proteoglycan having a molecular weight of not less than 1,800,000 (180×10⁴).
Item 2
   The water extract of fish cartilage according to Item 1, comprising a proteoglycan having a molecular weight of not less than 2,500,000 (250×10⁴).
Item 3
   The water extract of fish cartilage according to Item 2, comprising a proteoglycan having a molecular weight of not less than 5,000,000 (500×10⁴).
Item 4
   The water extract of fish cartilage according to Item 3, comprising, on a dry mass basis, 9 mass% or more of a proteoglycan having a molecular weight of not less than 5,000,000 (500×10⁴).
Item 5-1
   The water extract of fish cartilage according to any one of Items 1 to 4, comprising, on a dry mass basis, 30 mass% or more of collagen.
Item 5-2
   The water extract of fish cartilage according to any one of Items 1 to 5-1, wherein the proteoglycan having a molecular weight of not less than 2,500,000 (250 × 10⁴) contains uronic acid in an amount of not less than 10% based on the total amount of uronic acid in the extract.
Item 5-3
   The water extract of fish cartilage according to any one of Items 1 to 5-2, wherein the amount of uronic acid in the extract is, on a dry mass basis, not less than 10 mass% based on the entire extract.
Item 6-1
   The water extract of fish cartilage according to any one of Items 1 to 5, wherein the fish is salmon or trout.
Item 6-2
   The water extract of fish cartilage according to any one of Items 1 to 6-1, wherein the cartilage is nasal cartilage.
Item 7-1
   A composition for external use or an oral composition containing the water extract of fish cartilage according to any one of Items 1 to 6-2.
Item 7-2
   A food/drink composition, an oral composition, or a cosmetic composition containing the water extract of fish cartilage according to any one of Items 1 to 6-2.
Item 8-1
   The composition according to Item 7-1 or 7-2, for use in alleviation of inflammation or anti-aging.
Item 8-2
   The water extract of fish cartilage according to any one of Items 1 to 6-2, for use in alleviation of inflammation or anti-aging.
Item 9-1
   The water extract of fish cartilage according to any one of Items 1 to 6-2, wherein the water extract is extracted using 70°C to 100°C water.
Item 9-2
   The water extract of fish cartilage according to Item 9-1, wherein the water extract is extracted using 70°C to 100°C water for 3 to 4 hours.
Item 10-1
   An isolated proteoglycan having a molecular weight of not less than 5,000,000 (500×10⁴).
Item 10-2
   An isolated fish-cartilage-derived proteoglycan having a molecular weight of not less than 5,000,000 (500×10⁴).

### ■ Advantageous Effects of Invention

The fish cartilage water extract of the present invention contains proteoglycan of a molecular weight higher than that of the proteoglycan contained in the fish cartilage extracts obtained through hitherto known methods. Further, the fish cartilage water extract of the present invention has a superior anti-aging effect (in particular, anti-inflammatory effect, skin barrier functionality improving effect, and skin water-retention ability improving effect). Moreover, the fish cartilage water extract of the present invention provides these effects not only through application to skin, but also through oral administration.

### Brief Description of Drawings

Fig. 1a is a photo of a frozen salmon nasal cartilage block placed in a tray.
Fig. 1b is a photo of small pieces of frozen salmon nasal cartilage placed in a plastic bag.
Fig. 1c is a photo of defatted salmon nasal cartilage powder placed in a plastic bag.
Fig. 2 is a graph showing change in yield of proteoglycan over time when water was added to small pieces of frozen salmon nasal cartilage, and extraction was performed at 100°C. The yield is the value per gram of the frozen salmon nasal cartilage.
Fig. 3 is a graph showing a comparison of the yields of uronic acid (indicated as "GlcA") and the yields of protein (indicated as "Protein") when proteoglycan is extracted from defatted salmon nasal cartilage powder ("Powder"), frozen salmon nasal cartilage blocks ("Blocks"), or small pieces of frozen salmon nasal cartilage ("Small pieces"). The yields of GlcA and protein are represented in terms of amounts extracted per 100 g of frozen salmon nasal cartilage blocks. The amount of uronic acid reflects the amount of proteoglycan.
Fig. 4a shows a superimposition of a uronic acid amount chromatogram and a 280-nm protein amount chromatogram. The chromatograms were obtained by analyzing one example of a water extract of fish cartilage according to the present invention (Sample No. 10) by gel filtration chromatography.
Fig. 4b shows a uronic acid amount chromatogram with the position of each fraction at which each molecular weight marker was eluted. The chromatogram was obtained by analyzing one example of a water extract of fish cartilage according to the present invention (Sample No. 10) by gel filtration chromatography.
Fig. 5 shows a uronic acid amount chromatogram obtained by analyzing one example of a water extract of fish cartilage according to the present invention (Sample No. 10) by ion-exchange chromatography.
Fig. 6 shows a uronic acid amount chromatogram and a 280-nm protein amount chromatogram. The chromatograms were obtained by analyzing the proteoglycan fraction of Fig. 5 by gel filtration chromatography.
Fig. 7 shows the results of analyzing the inflammation alleviation effect obtained through oral administration of one example of a water extract of fish cartilage according to the present invention (Sample No. 10). The graph on the left shows the results after 4 weeks of UVB irradiation, and the graph on the right shows the results after 7 weeks of UVB irradiation.
Fig. 8 shows the results of analyzing the effect of enhancing and improving skin barrier functionality, obtained through oral administration of one example of a water extract of fish cartilage according to the present invention (Sample No. 10). The graph on the left shows the results after 4 weeks of UVB irradiation, and the graph on the right shows the results after 7 weeks of UVB irradiation.
Fig. 9 shows the results of analyzing the inflammation alleviation effect obtained through oral administration of Sample No. 10, PG-1 extract, and PG-2 extract. PG-1 extract and PG-2 extract were obtained by further purifying Sample No. 10. The graph on the left shows the results after 4 weeks of UVB irradiation, and the graph on the right shows the results after 7 weeks of UVB irradiation.
Fig. 10 shows the results of analyzing the effect of enhancing and improving skin barrier functionality, obtained through oral administration of Sample No. 10, PG-1 extract, and PG-2 extract. PG-1 extract and PG-2 extract were obtained by further purifying Sample No. 10. The graph on the left shows the results after 4 weeks of UVB irradiation, and the graph on the right shows the results after 7 weeks of UVB irradiation.
Fig. 11 shows the results of analyzing the effect of enhancing and improving skin water retention ability, obtained through oral administration of Sample No. 10, PG-1 extract, and PG-2 extract. PG-1 extract and PG-2 extract were obtained by further purifying Sample No. 10. The graph on the left shows the results after 4 weeks of UVB irradiation, and the graph on the right shows the results after 7 weeks of UVB irradiation.
Fig. 12 shows the results of a clinical test for analyzing the effect of enhancing and improving skin barrier functionality, obtained through oral administration of Sample No. 10.
Fig. 13 shows the results of a clinical test for analyzing the effect of enhancing and improving skin water retention ability, obtained through oral administration of Sample No. 10.
Fig. 14 shows the results of a cell proliferation test when extracts extracted at heating temperatures of 50°C, 70°C, 90°C, 100°C, and 100°C (extra heating time), and commercially available product A, were individually added. The asterisk (*) refers to significance at significance level of 0.1% relative to the control.

### Description of Embodiments

The present invention is described in more detail below. The term "mass" in this specification is equivalent to "weight".

Proteoglycan has a structure in which glycosaminoglycan (mucopolysaccharide) and protein are covalently bonded. Glycosaminoglycan is acidic saccharide consisting of a repeating disaccharide unit. Specifically, examples thereof include chondroitin sulfate, dermatan sulfate, and heparan sulfate. In the repeating disaccharide units of the acidic saccharide components, generally, one of the disaccharides is amino sugar, and the other is a uronic acid. Therefore, the detection of proteoglycan may be performed using a carbazole-sulfuric acid method, which is an ordinary method for detecting uronic acids.

Further, a compound having a comb-like bond of glycosaminoglycan and protein is referred to as a proteoglycan monomer. (This protein in a proteoglycan monomer is called a core protein.) In particular, in a living body, the proteoglycan monomer is bonded to a hyaluronic acid via a link protein, thereby forming an aggregate. The aggregate is also called a proteoglycan aggregate. The term "proteoglycan" used in this specification encompasses a proteoglycan monomer and a proteoglycan aggregate. Hyaluronic acids are a kind of glycosaminoglycan.

The fish cartilage water extract of the present invention contains a high molecular weight proteoglycan. The term "high molecular weight proteoglycan" used herein specifically refers to a proteoglycan having a molecular weight of not less than 1,800,000 (180 × 10⁴), preferably a molecular weight of not less than 2,500,000 (250 × 10⁴), not less than 3,500,000 (350 × 10⁴), not less than 4,000,000 (400 × 10⁴), not less than 5,000,000 (500 × 10⁴), not less than 6,000,000 (600 × 10⁴), not less than 7,000,000 (700 × 10⁴), not less than 8,000,000 (800 × 10⁴), not less than 9,000,000 (900 × 10⁴), not less than 10,000,000 (1000 × 10⁴), not less than 11,000,000 (1100 × 10⁴), not less than 12,000,000 (1200 × 10⁴), not less than 13,00,000 (1300 × 10⁴), not less than 14,000,000 (1400 × 10⁴), not less than 15,000,000 (1500 × 10⁴) not less than 16,000,000 (1600 × 10⁴), not less than 17,000,000 (1700 × 10⁴), not less than 18,000,000 (1800 × 10⁴), not less than 19,000,000 (1900 × 10⁴), or not less than 20,000,000 (2000 × 10⁴). A greater molecular weight is preferable. Particularly preferred is a proteoglycan having a molecular weight of not less than 5,000,000 (500 × 10⁴). The fish cartilage water extract of the present invention is subjected to gel filtration chromatography under the following conditions, and the uronic acid amount (reflecting a proteoglycan amount) in each fraction is determined using a carbazole-sulfuric acid method. By creating a chromatogram based on the determined uronic acid amounts, it is possible to confirm the existence of proteoglycan having the above range of molecular weight. Such a chromatogram based on the uronic acid amount may be hereinafter referred to as "uronic acid amount chromatogram". Further, it is also possible to make a chromatogram based on the absorbencies by measuring absorbencies of the fractions at 280 nm, and then finding relative values of protein amounts based on the measurement results (i.e., the measurement values are assumed to be values that reflect the protein amounts). Hereinafter, such a chromatogram may be referred to as "280 nm protein amount chromatogram".

### Gel Filtration Chromatography Conditions

Column: Sepharose CL-2B packed column (1-cm dia. × 50 cm column packed with Sepharose CL-2B as a carrier. Sepharose CL-2B has a dextran fractionation range of 100 to 20,000 kDa and is available from GE Healthcare and other companies. Sepharose CL-2B, CAS registry No. 65099-79-8, is a 2% crosslinked agarose with a particle size of 60 to 200 µm (measured by the laser diffraction scattering method).)
Buffer: 0.1 M phosphate buffer (pH of 7.1, containing 0.2 M NaCl) Amount of applied sample: 4 mg of water extract of fish cartilage (on a dry mass basis) (dissolved in 1 mL of buffer for use)
Flow rate: 0.15 mL/min
Amount of fraction: 1 mL/tube
Molecular weight analytical curve: The various dextran molecular weight markers described below are subjected to gel filtration chromatography under the same conditions as described above, absorbency (which reflects the amount of dextran) of each fraction is measured by the phenol-sulfuric acid method, which is a well-known method for detecting saccharide, a fraction at which each marker is eluted is determined, and an analytical curve reflecting the molecular weight of the component contained in each fraction in the gel filtration chromatography under the above conditions is prepared. The term "fraction at which each marker is eluted" refers to a fraction at which each marker is eluted most, i.e., a fraction corresponding to a peak maximum in a chromatogram reflecting the amount of dextran when each dextran molecular weight marker is subjected to gel filtration.

### Dextran Molecular Weight Markers

Dextran from *Leuconostoc mesenteroides* (mol wt 5,000,000 - 40,000,000)(Sigma) ... For measuring the void volume of the column, 20,000 kDa
Dextran Standard 1,400,000 (Sigma) ... 1400 kDa
Dextran Standard 670,000 (Sigma) ... 670 kDa
Dextran Standard 410,000 (Sigma) ... 410 kDa
Dextran Standard 270,000 (Sigma) ... 270 kDa

The dextran from *Leuconostoc mesenteroides* is used as a marker after pretreatment is carried out to remove low molecular weight dextran contained in the marker. The pretreatment is performed by eluting dextran from *Leuconostoc mesenteroides* under the conditions described above in "Gel Filtration Chromatography Conditions" and collecting and freeze-drying fractions having a molecular weight of not less than 20,000 kDa. More specifically, fractions that correspond to a peak that first appears in a chromatogram reflecting the amount of dextran are collected and freeze-dried. The chromatogram is prepared by measuring absorbency of each fraction by the phenol-sulfuric acid method. (It is believed that dextran having a molecular weight of not less than 20,000 kDa is obtained by collecting and freeze-drying such fractions.) This freeze-dried product is actually used as a marker (for measuring the void volume of the column).

Measurement of absorbency to obtain a chromatogram reflecting the amount of dextran is performed according to the method (the phenol-sulfuric acid method) described in Hodge, J. E., and Hofreiter, B. T., Method in Carbohydrate Chemistry, 1, 338 (1962). More specifically, the measurement is carried out as follows.
[1] 500 µL of a sample aqueous solution is placed in a 105 × 15 mm test tube.
[2] 500 µL of a phenol reagent (5 v/v% aqueous phenol solution) is added thereto, and the mixture is stirred.
[3] 2.5 mL of concentrated sulfuric acid is added thereto, and immediately the mixture is stirred vigorously for 10 seconds.
[4] The mixture is left to stand for 20 minutes or more at room temperature.
[5] The absorbency at 490 nm is measured with a spectrophotometer.

The carbazole-sulfuric acid method refers to a well-known method performed by adding a carbazole solution, which is a color component of uronic acid (glucuronic acid (Glc A), iduronic acid, etc.), to a measurement specimen, and measuring the absorbency by using a spectrophotometer, thereby finding uronic acid amount based on the absorbency. An analytical curve is plotted using the glucuronic acid standard solution having a specific concentration, thereby finding the glucuronic acid content in the specimen. More specifically, the carbazole-sulfuric acid method is performed as follows. 2.5 ml of a reagent obtained by dissolving 0.95 g of sodium borate decahydrate in 100 ml of a concentrated sulfuric acid is placed in a test tube and ice-cooled. 0.5 ml of a test object (preferably containing 2 to 20 µg of uronic acid) is gently layered thereon. The mixture is stirred well while being ice-cooled, thereby keeping it at room temperature or below. After the test tube is covered with a glass ball, the test tube is heated in a boiling water bath for 10 minutes, followed by water cooling to decrease the temperature to room temperature. Then, 0.1 ml of a reagent obtained by dissolving 125 mg of carbazole in 100 ml of anhydrous methyl alcohol is added and mixed therewith, and the mixture is heated in a boiling water bath for 15 minutes. Thereafter, the mixture is water-cooled to room temperature, and the absorbency at 530 nm is measured. In the blank test, 0.5 ml of distilled water is used. Simultaneously, an analytical curve is plotted using a glucuronic acid. (The carbazole-sulfuric acid method in the Examples described below is performed in the same manner as above.)

It is preferable that, on a dry mass basis, at least 10 mass% of the total amount of the uronic acid amount (determined by the carbazole-sulfuric acid method) of the fish cartilage water extract of the present invention is derived from proteoglycan having a molecular weight of not less than 2,500,000 (250 × 10⁴). In other words, on a dry mass basis, the uronic acid amount of a proteoglycan having a molecular weight of not less than 2,500,000 (250 × 10⁴) is preferably at least 10 mass% based on the total uronic acid amount of the fish cartilage water extract of the present invention. Further, the uronic acid amount is more preferably not less than 15 mass%, not less than 20 mass%, not less than 25 mass%, not less than 30 mass%, not less than 35 mass%, not less than 40 mass%, not less than 45 mass%, not less than 50 mass%, not less than 55 mass%, or not less than 60 mass%. The greater the mass%, the more desirable. Further, a uronic acid amount of proteoglycan having a molecular weight of not less than 5,000,000 (500 × 10⁴) is preferably at least 7 mass% based on the total uronic acid amount of the fish cartilage water extract of the present invention. Further, the uronic acid amount of proteoglycan having a molecular weight of not less than 5,000,000 (500 × 10⁴) is more preferably not less than 10 mass%, not less than 13 mass%, not less than 16 mass%, not less than 20 mass%, not less than 24 mass%, not less than 27 mass%, not less than 30 mass%, not less than 34 mass%, not less than 37 mass%, or not less than 40 mass%. The greater the mass%, the more desirable.

The proportion of the uronic acid amount of a proteoglycan having a molecular weight of not less than a specific value (which is expressed as X hereinbelow) based on the total uronic acid amount in the extract can be found from a peak area in the aforementioned uronic acid amount chromatogram. More specifically, the proportion can be found by calculating a proportion of the area of a uronic acid having a molecular weight of not less than X based on the entire peak area of the uronic acid amount chromatogram. Even more specifically, on a uronic acid amount chromatogram in which the vertical axis denotes uronic acid amounts and the horizontal axis denotes the fraction number, a vertical line is drawn so that the vertical line passes through the fraction containing the proteoglycan having a molecular weight X; and then, from the two peak areas divided by the vertical line, a peak area containing proteoglycan of a greater molecular weight is determined and the proportion of the peak area based on the total peak area is found.

Further, the uronic acid amount (measured by the carbazole-sulfuric acid method) contained in the fish cartilage water extract of the present invention is, on a dry mass basis, preferably not less than 5 mass%, more preferably not less than 10 mass%, even more preferably not less than 15 mass%, still more preferably not less than 20 mass%, particularly preferably not less than 25 mass%. In the present specification (in particular, figures and tables), the term "GlcA" may be used as an abbreviation for glucuronic acid (e.g., "GlcA(µg)")

Further, the fish cartilage water extract of the present invention preferably contains proteoglycan having a molecular weight of not less than 1,800,000 (180 × 10⁴) in an amount of, on a dry mass basis, not less than 30 mass%, more preferably not less than 35 mass% of the extract.

Further, the fish cartilage water extract of the present invention preferably contains proteoglycan having a molecular weight of not less than 5,000,000 (500 × 10⁴) in an amount of, on a dry mass basis, not less than 5 mass%, not less than 10 mass%, not less than 15 mass%, not less than 20 mass%, not less than 25 mass%, not less than 30 mass%, or not less than 35 mass% of the extract. A greater proportion is more desirable.

Further, the fish cartilage water extract of the present invention contains only a small amount of lipid. The mass ratio of lipid to proteoglycan (lipid amount/proteoglycan amount) in the fish cartilage water extract of the present invention is preferably smaller than the mass ratio of lipid to proteoglycan (lipid amount/proteoglycan amount) in fish cartilage used as the raw material of the extract. The lipid content in the fish cartilage water extract of the present invention is, on a dry mass basis, preferably not more than 5 mass%, more preferably not more than 4 mass%, even more preferably not more than 3 mass%, still more preferably not more than 2 mass%, particularly preferably not more than 1 mass%. As described below, such a fish cartilage water extract can be obtained by using defatted fish cartilage (fish cartilage from which lipid is removed). Further, the fish cartilage water extract may also be obtained by defatting the resulting extract using a known defatting method.

Further, most of the protein contained in fish cartilage extract of the present invention is preferably collagen (preferably type II collagen). More specifically, preferably not less than 80 mass%, more preferably not less than 85 mass% of the protein is collagen. The amount of collagen in the fish cartilage extract of the present invention is, on a dry mass basis, preferably not less than 30 mass%, more preferably 30 to 60 mass%.

Further, the ash content in the fish cartilage extract of the present invention is, on a dry mass basis, preferably not more than 15 mass%, more preferably 5 to 15 mass%, even more preferably 5 to 10 mass%.

The protein amount above is found by measuring the nitrogen amount in the sample using the Kjeldahl method, and subtracting the glycosaminoglycan-derived nitrogen amount from the above nitrogen amount; i.e., the protein amount is the amount excluding the amount of proteoglycan-derived protein. Further, the ash content is found by the direct ashing method. Further, the collagen amount is found by measuring the hydroxy proline amount in the sample using the automatic amino acid analysis (HPLC method), and calculating the collagen amount assuming that the collagen contains 6.7% hydroxy proline.

The fish cartilage water extract of the present invention is extracted from fish cartilage, preferably from *Oncorhynchus* (*Salmonidae*) cartilage, including trout (humpback salmon, cherry salmon, satsukimasu salmon, etc.), salmon (chum salmon, sockeye salmon, silver salmon, chinook salmon, steelhead, etc.), shark, and cod. Salmon and trout are particularly preferable. The cartilage to be used is not particularly limited; however, head cartilage, in particular, nasal cartilage, is preferable. Moreover, since fish (in particular, salmon and trout) heads are usually discarded when fish is processed into food products, the cost of fish heads is low, and a large amount of fish heads can be stably supplied.

The extraction is performed using water. It is possible to subject fish cartilage obtained from a biological sample to extraction as it is; however, the fish cartilage may also be subjected to extraction after being pulverized (more specifically, pulverized into small pieces or powder). Further, as described below, it is also possible to subject fish cartilage to a defatting treatment using ethanol or the like before the extraction. As such, proteoglycan (including a high molecular weight proteoglycan) can be extracted using water. More specifically, the fish cartilage water extract of the present invention is preferably extracted from fish cartilage by using water. It is also possible to perform water extraction while heating water, or by using hot or boiling water. With this method, a fish cartilage water extract with a greater effect can be efficiently obtained.

The small pieces of fish cartilage are obtained by pulverizing fish cartilage into small pieces. The pulverization may be performed using a known method. For example, the pulverization of fish cartilage (preferably frozen fish cartilage) into small pieces may be performed using known devices such as a blender or a mill. The pulverization is preferably performed at a low temperature. For example, the pulverization is preferably performed at a temperature at which the pulverized fish cartilage can be kept frozen. More specifically, the pulverization is preferably performed at a temperature of 0°C or less.

Further, in terms of efficiency in extraction, it is preferable to use small frozen pieces of fish cartilage. The small frozen pieces of fish cartilage can be obtained by (i) freezing fish cartilage and then pulverizing it into small pieces, or by (ii) pulverizing fish cartilage into small pieces and then freezing it. The small frozen pieces of fish cartilage obtained by method (i) are more preferable. The freezing method is not particularly limited, and any known freezing method can be used. For example, a method of storing fish cartilage in a freezer at about -20 to -80°C for about 24 to 72 hours can be used.

The small pieces of fish cartilage or small frozen pieces of fish cartilage are preferably about 0.001 to 0.5 g, more preferably about 0.005 to 0.3 g, even more preferably about 0.01 to 0.1 g per piece. The pulverization of fish cartilage into small pieces is preferably performed in a manner enabling production of such small pieces. (The conditions for obtaining such small pieces can be easily obtained by appropriately selecting and adjusting the device to be used.)

The fish cartilage powder is obtained by pulverizing fish cartilage into powder (powdered fish cartilage). The pulverization may be performed using a known method. For example, the pulverization of fish cartilage (preferably frozen fish cartilage) into powder may be performed using known devices such as a blender or a mill. The pulverization is preferably performed at a low temperature (e.g., not more than 0°C).

Further, in terms of efficiency in extraction, it is preferable to use frozen fish cartilage powder. The frozen fish cartilage powder can be obtained by (i') freezing fish cartilage and then pulverizing it into powder, or by (ii') pulverizing fish cartilage into powder and then freezing it. The frozen fish cartilage powder obtained by method (i') is more preferable. The freezing method is not particularly limited, and any known freezing method can be used. For example, a method of storing fish cartilage in a freezer at about -20 to -80°C for about 24 to 72 hours can be used.

The term "powder" refers to a piece smaller than what is referred to by the term "small pieces"; however, these terms are not necessarily distinguished clearly. Among the products resulting from pulverization of fish cartilage, relatively large pieces are referred to as "small pieces", and relatively small pieces are referred to as "powder". Therefore, although it is not particularly limited, powder preferably contains particles having a particle size of about 10 to 1000 µm, preferably about 50 to 500 µm, more preferably about 100 to 200 µm (measured by a laser diffraction and scattering method). However, the particle size is not particularly limited to these ranges. The powder preferably contains particles having the above particle size in a large proportion (e.g., not less than 50 mass%, preferably not less than 70 mass%).

Small defatted pieces of fish cartilage or fish cartilage powder (small fish cartilage pieces or powder from which lipid is removed) may also be used. In other words, small defatted fish cartilage pieces or defatted fish cartilage powder may also be used. By using defatted fish cartilage, a highly purified fish cartilage water extract that incorporates less lipid can be obtained. The small defatted fish cartilage pieces or defatted fish cartilage powder can be obtained by (α) pulverizing defatted fish cartilage into small pieces or powder, or by (β) pulverizing the fish cartilage into small pieces or powder and then defatting the small pieces or powder.

The defatting may be performed by a known method. For example, fish cartilage defatting step (α) above may be performed by a method of running fish cartilage under water (e.g., tap water) for about 1 to 24 hours. Preparation of fish cartilage can be performed using a known method, including a method of immersing fish tissues (preferably a fish head) in water for about 1 to 24 hours to make the tissue swell, and removing tissues other than cartilage (preferably nasal cartilage), and a method of thawing a frozen salmon head, then immediately separating the nasal cartilage and running the nasal cartilage under water for about 1 to 24 hours, thereby washing and defatting the cartilage. If the cartilage has residual flesh, it is preferable to remove the flesh with tweezers or the like. At this point, since the fish cartilage has not been pulverized into small pieces or powder, it is likely that little proteoglycan will be extracted even after running the cartilage under water. Further, as in step (β) below, lipid can be removed using an organic solvent.

Further, for example, the method for defatting the small fish cartilage pieces or fish cartilage powder in step (β) above may be performed by extracting and removing lipid using an organic solvent. Examples of the organic solvents include ethanol, hexane, acetone, and the like. More specifically, as step (β), a method disclosed in Patent Document 1 (JP2009-173702A) can be preferably used. More specifically, for example, defatted fish cartilage powder, which is obtained by a method including the following steps A to E, can be preferably used for the present invention. (Patent Document 1 also discloses more detailed conditions.)
A. Frozen aquatic animal tissues (fish tissues) are crushed and treated at 0 to 20°C, pH of 4.8 to 7, after adding water.
B. A solid-liquid mixture obtained by step (A) is centrifuged to remove the uppermost lipid layer and the middle aqueous layer, thereby collecting a precipitate.
C. The precipitate is dried and pulverized into powder.
D. A solvent, namely, hexane, acetone or ethanol, is added to the obtained dried powder to extract and remove residual lipid.
E. The solvent is removed.

In addition, it is more preferable to use small frozen and defatted fish cartilage pieces or fish cartilage powder (small frozen and defatted fish cartilage pieces or frozen and defatted fish cartilage powder). These can be obtained, for example, by freezing defatted fish cartilage and pulverizing the frozen fish cartilage into small pieces or powder.

The fish cartilage, preferably small fish cartilage pieces, or fish cartilage powder (which hereinafter may be referred to collectively as "fine fish cartilage"), is subjected to water extraction. Examples of water used for water extraction (which hereinafter may be referred to as "extraction water") include Milli-Q water, distilled water, deionized water, purified water, and tap water. Further, the pH of the extraction water is generally about 5.5 to 8.0, preferably about 6.0 to 7.5, more preferably about 6.5 to 7.5. It is not preferable to dissolve substances that greatly change the pH, such as acids, alkalis, bases, and the like. If an acid compound such as organic acid or inorganic acid, or an alkali compound such as sodium hydroxide, is added to the extraction water, a high molecular weight proteoglycan (in particular, a high molecular weight proteoglycan having a molecular weight of 10,000,000 (1000 × 10⁴)) is reduced or disappears; thus, it is not preferable to add acid compounds or alkali compounds. Although a restrictive interpretation is not desired, this is presumably because acid compounds and alkali compounds cause degradation of proteoglycan aggregates during extraction.

The water extract can be obtained, for example, by immersing fish cartilage in water for an appropriate amount of time (e.g., not less than 30 minutes, preferably about 30 minutes to 24 hours, more preferably about 1 to 12 hours, even more preferably about 2 to 6 hours, still more preferably about 3 to 4 hours). The amount of water is not particularly limited; for example, the water amount is a sufficient amount for completely immersing all the small fish cartilage pieces or fish cartilage powder subjected to extraction in water. The fish cartilage pieces or powder may be allowed to stand still; more preferably it may be stirred. Further, the water temperature during the extraction is not particularly limited; however, the temperature is preferably not less than 50°C, more preferably not less than 70°C. To ensure this temperature range, the water may be heated during the extraction or before the extraction. The heating temperature (i.e., the temperature of the water used) is preferably about 50 to 100°C, more preferably about 70 to 100°C, even more preferably about 80 to 100°C. The heating may be performed under an increased pressure. Since heating may cause degradation of high molecular weight proteoglycan, the heated extraction water may be replaced during the extraction. The interval between replacements of extraction water is, for example, every 15 minutes to 4 hours, preferably every 30 minutes to 2 hours, or about 1 hour. As a preferable method, a method of adding water (preferably heated water) to fish cartilage in a sufficient amount for completely immersing the total amount of fish cartilage, and allowing it to stand or stirring it for 3 to 4 hours while heating may be used. Another preferable method is repeatedly performing a process of adding water (preferably heated water) to fish cartilage in a sufficient amount for completely immersing the total amount of fish cartilage, allowing it to stand for an hour while heating, and collecting the resulting water. (In this case, the water extraction takes 4 hours in total.)

After the water extraction, the liquid portion is collected to obtain a fish cartilage water extract. The collection of the liquid portion is performed by removing the supernatant through, for example, a centrifugation treatment (e.g., centrifugation at 5000 rpm, 4°C, for 20 minutes) or a continuous centrifugation treatment. The liquid (supernatant) may be used, as it is, as the fish cartilage water extract of the present invention, or it may further be purified by a known method (e.g., defatting). It is also possible to concentrate the liquid by distillation under reduced pressure or the like. It is also possible to dry or powderize the liquid according to the freeze-drying method or the spray drying method. The fish cartilage water extract of the present invention thus obtained may be used as, for example, materials for food, cosmetics, oral compositions, medicinal products, and the like.

The fish cartilage water extract of the present invention may be suitably used for, in particular, skin anti-aging. Skin is constantly exposed to various kinds of damage. For example, in the dermal layer, the epidermis barrier functionality decreases due to external factors (e.g., light irradiation such as ultraviolet) or internal factors (e.g., aging). Such damage causes an increase in transepidermal water loss (TEWL), thereby causing dry skin, rough skin or the like. Further, for the same reason, a decrease in the ability to produce collagen, a decrease in skin elasticity, or thickening of the dermis may occur in the dermal layer, thereby furthering hardening of the skin. This may result in wrinkles or the like.

The fish cartilage water extract of the present invention exhibits various effects (anti-inflammatory effect, skin barrier functionality improving effect, skin water-retention ability improving effect, and cell proliferation promoting effect) to inhibit or alleviate such skin symptoms. In particular, the fish cartilage water extract of the present invention is suitable to prevent or alleviate these skin symptoms caused by light irradiation (exposure) (in particular, ultraviolet irradiation). The degree of the cell proliferation promoting effect varies depending on the temperature during water extraction. This is presumably because the temperature change during water extraction changes the composition of the fish cartilage water extract.

The usage of the fish cartilage water extract of the present invention is not limited; however, since the fish cartilage water extract of the present invention provides the aforementioned effects through embrocation or oral administration, the fish cartilage water extract of the present invention is suitable for compositions for external use or oral compositions. More specifically, the fish cartilage water extract of the present invention is preferably used as a composition for external use or an oral composition. These compositions may be used as a medicinal composition, a quasi-drug composition, an oral composition, a cosmetic composition, a food composition, and the like. They may be produced by a standard method using the fish cartilage water extract of the present invention. They are particularly useful for products in the oral-care industry, skin-care industry, hair-care industry, and health-care industry.

The oral compositions containing the fish cartilage water extract of the present invention (which may be hereinafter referred to as oral compositions of the present invention) used in the oral-care industry may be the fish cartilage water extract of the present invention itself, or a composition produced by appropriately combining the fish cartilage water extract of the present invention with other components (e.g., abrasives, binders, surfactants, excipients, wetting agents, pH adjusters, thickeners, sweetening agents, preservatives, medicinal components, coloring agents, flavoring agents, and the like) generally used for oral compositions. Examples of the oral composition products include paste agents, ointments, gels, embrocations, sprays, supplements, liquids, mouthwashes, pastes, chewing gum, troches, and tablets, which may be manufactured by standard methods.

Such oral compositions of the present invention can be preferably used for alleviation of inflammation in oral tissues, anti-aging in oral tissues, and prevention or alleviation of dryness of the oral tissues. More specifically, the oral compositions of the present invention encompass an inflammation alleviation oral composition, an anti-aging oral composition, a tissue-repairing oral composition, and an oral dryness prevention/alleviation oral composition.

The cosmetic composition (hereinafter may be referred to as "cosmetic composition of the present invention") containing the fish cartilage water extract of the present invention used as hair-care products or skin-care products may be the fish cartilage water extract of the present invention itself, or a composition produced by appropriately combining the fish cartilage water extract of the present invention with cosmetically acceptable surfactants, wetting agents, polymeric agents, powder, esters, alcohols, plant or animal oils, medicinal agents, preservatives, coloring agents, flavoring agents, or other cosmetically acceptable components or materials using a standard method. More specifically, the cosmetic compositions produced by incorporating the fish cartilage water extract of the present invention include hair restorers, hair tonics, emulsions, lotions, creams, serums, face packs, face masks, and sunscreens. Such a cosmetic composition of the present invention may be preferably used for alleviation of inflammation, anti-aging, or moisture retention. Examples of preferable usages include compositions for hair restoration, scalp care, sun protection, sunburn care, moisturizing and anti-aging of the skin (e.g., prevention or alleviation of dry skin, rough skin, facial wrinkles, or sagging skin).

The food and beverage compositions (beverages and food) containing the fish cartilage water extract of the present invention (which may be hereinafter referred to as food and beverage compositions of the present invention) used in the health-care industry may be the fish cartilage water extract of the present invention itself, or a composition produced by appropriately combining the fish cartilage water extract of the present invention with bases, carriers or additives that are acceptable in terms of food hygiene, or other components or materials that are used for food and beverages. Examples of these include processed food and beverages containing the fish cartilage water extract of the present invention with claimed effects of moisturizing and anti-aging of the skin (e.g., prevention or alleviation of dry skin, rough skin, facial wrinkles, or sagging skin), dietary supplements, food for special purposes (food for patients, food for specific health uses, food for patients deglutition disorder, etc.) and beauty food. Moreover, the present invention also includes moisturizers and skin anti-aging agents formed of the aforementioned food and beverage compositions of the present invention. The moisturizers and skin anti-aging agents may be supplied in the forms of drinks, pills, tablets, capsules, granules, jelly, troches, pastes, viscous liquids, powder, block bars, or the like for cosmetic or skin anti-aging purposes (e.g., prevention or alleviation of dry skin, rough skin, facial wrinkles, or sagging skin).

The amount of the fish cartilage water extract contained in the compositions for external use or oral composition (in particular, oral compositions, cosmetic compositions, or food or beverage compositions of the present invention) containing the fish cartilage water extract of the present invention is, for example, but not limited to, generally 0.0001 to 100 mass%, preferably 0.001 to 50 mass%, more preferably 0.005 to 30 mass%, even more preferably 0.01 to 20 mass%, based on the entire composition. Further, the amount of uronic acid derived from proteoglycan having a molecular weight of not less than 2,500,000 (250 × 10⁴) in the composition is, for example, about 0.00001 to 10 mass%, preferably about 0.0001 to 5 mass%, more preferably about 0.0005 to 1 mass%, based on the entire composition.

Further, the present invention also encompasses methods for obtaining the various effects disclosed in this specification, including a method for alleviating inflammation on the skin or in the oral cavity, a method for enhancing or improving barrier functionality, and a method for promoting cell (in particular, fibroblast) proliferation, by applying the fish cartilage water extract of the present invention to skin or the oral cavity. These methods may be performed using the fish cartilage water extract of the present invention as it is, more preferably, using the above compositions for external use, or oral compositions or cosmetic compositions of the present invention. The target subject of the method is not particularly limited; however, the method is preferably used for subjects having inflammation or subjects having decreased skin barrier functionality due to aging or sunburn. The method may also be used not only for treatment purposes but also for cosmetic purposes. The method is particularly suitable for cosmetic purposes for enhancing barrier functions. The target subjects are preferably mammals, including humans. More specifically, non-human mammals can also be target subjects. Examples of non-human mammals include pet animals or livestock, including dogs, cats, apes, mice, rats, hamsters, rabbits, cows, horses, swine, sheep, goats, Ilamas, and camels. The application amount is also not limited, and a suitable effective amount can be determined. In particular, in application to humans, it is preferable to adjust the dosage of the fish cartilage water extract by adjusting the amount of proteoglycan, for example, so that the amount of the uronic acid derived from a high molecular weight proteoglycan having a molecular weight of not less than 2,500,000 (250 × 10⁴) (more preferably, not less than 5,000,000 (500 × 10⁴)) is preferably about 0.1 to 500 mg, more preferably about 0.5 to 250 mg, even more preferably about 1 to 100 mg, per adult/day.

Furthermore, the present invention also encompasses methods for obtaining the various effects disclosed in this specification, including a method for alleviating inflammation on the skin or in the oral cavity, a method for enhancing or improving skin barrier functionality, a method for inhibiting thickened skin and a method for improving skin water retention ability, through oral administration of the fish cartilage water extract of the present invention. These methods may be performed using the fish cartilage water extract of the present invention as it is, more preferably, using the oral compositions described above, or food and beverage compositions of the present invention. Further, medicinal compositions containing the fish cartilage water extract of the present invention are also preferably used. The target subject of the method is not particularly limited; however, the method is preferably used for subjects who developed inflammation, or subjects with decreased skin barrier functionality or skin water retention ability due to aging or sunburn. The method may also be used for cosmetic purposes. The target subjects are preferably mammals including humans. More specifically, non-human mammals can also be target subjects. Examples of non-human mammals include pet animals or livestock, including dogs, cats, apes, mice, rats, hamsters, rabbits, cows, horses, swine, sheep, goats, Ilamas, and camels. The application amount is also not limited, and a suitable effective amount can be determined. In particular, in the application to humans, it is preferable to adjust the dosage of the fish cartilage water extract by adjusting the amount of proteoglycan, for example, so that the amount of the uronic acid derived from the high molecular weight proteoglycan having a molecular weight of not less than 2,500,000 (250 × 10⁴) (more preferably, not less than 5,000,000 (500 × 10⁴)) is about 0.2 to 1000 mg, more preferably about 1 to 800 mg, even more preferably about 2 to 500 mg, per adult/day.

### Examples

The present invention is described below in more detail. However, the scope of the invention is not limited to these Examples.

### Analysis of Extraction of Proteoglycan from Salmon Nasal Cartilage

### Samples Used for Extraction

The following three types ((1) to (3)) of samples derived from salmon nasal cartilage were used for analyzing extraction of proteoglycan. Salmon nasal cartilage used for the analysis was obtained by separating nasal cartilage immediately after thawing a frozen salmon head, washing and defatting the nasal cartilage by running it under water for 6 hours, removing pieces of flesh and the like with tweezers, and washing the nasal cartilage with water by hand.

### (1) Frozen Salmon Nasal Cartilage Blocks

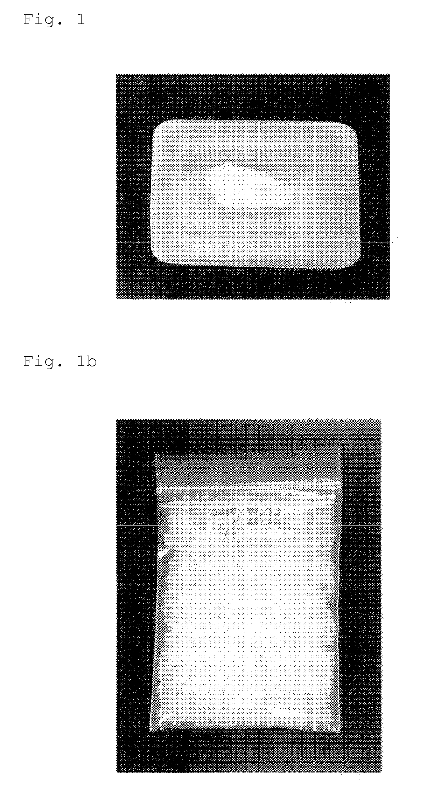

Salmon nasal cartilage was stored and frozen in a freezer, and the frozen nasal cartilage was used as a frozen salmon nasal cartilage block. The frozen salmon nasal cartilage block had a size of about 2.5 × 1.5 cm to about 4.5 × 2 cm and a weight of about 1.71 g to about 6.91 g. (The average weight of 7 blocks was 3.701 g.), although the size and weight depend on the size of the salmon head used. Fig. 1a shows a photo of a frozen salmon nasal cartilage block.

### (2) Small Pieces of Frozen Salmon Nasal Cartilage

Frozen salmon nasal cartilage blocks from item (1) above were placed in a blender and crushed for 10 seconds to prepare small pieces of frozen salmon nasal cartilage. Fig. 1b shows a photo of small pieces of frozen salmon nasal cartilage. Twenty pieces were randomly collected, and the size and the mass of each piece were analyzed. Each piece had a size of about 0.2 cm to about 0.7 cm and a mass of about 0.0116 to about 0.0890 g. (The average weight of 20 pieces was 0.033 g.) From 100 g of the frozen salmon nasal cartilage blocks, 95.6 g of small pieces of frozen salmon nasal cartilage was obtained.

### (3) Defatted Salmon Nasal Cartilage Powder

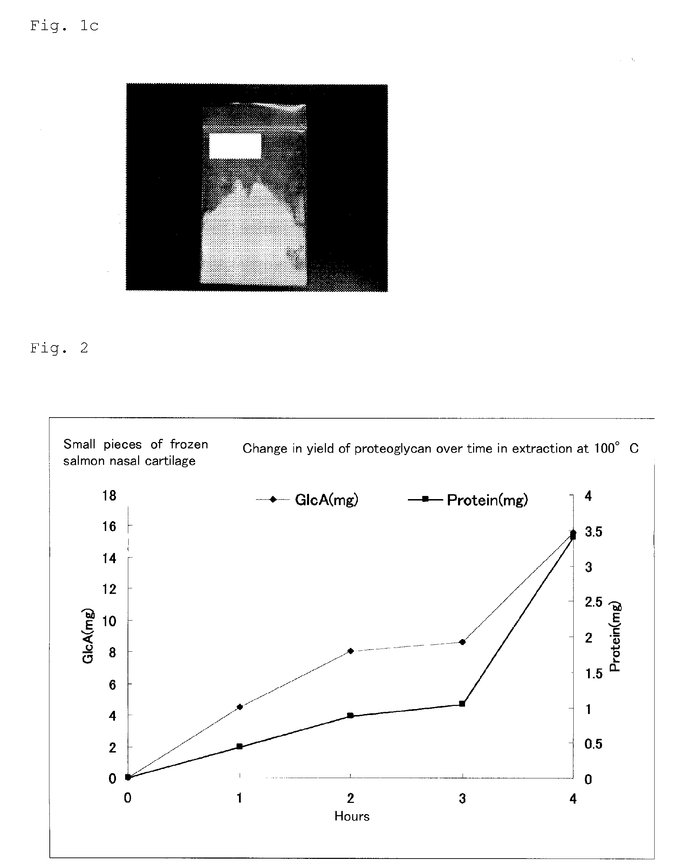

Defatted salmon nasal cartilage powder was prepared using frozen salmon nasal cartilage from item (1) above by the method disclosed in Example 1 of Patent Document 1 (JP2009-173702A). From 100 g of the frozen salmon nasal cartilage blocks, 5.83 g of defatted salmon nasal cartilage powder was obtained. Fig. 1c shows a photo of defatted salmon nasal cartilage powder.

The following is an excerpt from the disclosure of Example 1 of Patent Document 1.
"Frozen salmon nasal cartilage (100 g) was crushed, and an equal volume of tap water at 15°C was added to the crushed salmon nasal cartilage. The mixture was gently stirred to mix it thoroughly, and the mixture, which was maintained at about 5°C, was immediately centrifuged with a centrifugal separator at 9,000 rpm at 4°C for 30 minutes to separate lipid and other components including proteoglycan. Three layers were obtained after the centrifugation. The lipid layer in the upper layer and the aqueous layer in the middle layer were removed, and the precipitate was collected. The precipitate was freeze-dried and then pulverized with a centrifugal mill to prepare a water-defatted fine powder. At this stage, some of the fine powder was subjected to ether extraction for measurement of lipid, and it was found that 8.8% lipid remained, that the removal rate was 75.0% when lipid before defatting was defined as 100%, and that the lipid had faint foul odor. Subsequently, a 10-fold volume of ethanol was added to the water-defatted fine powder to dissolve and extract the lipid with the foul odor. This operation was repeated twice. The ethanol solution was filtered off and the solvent was evaporated to obtain a pale yellow-brown, odorless proteoglycan composition powder. The yield relative to the salmon nasal cartilage was 58.7% (dry basis), and the proteoglycan content was 77.7%. The foul odor of the proteoglycan composition powder completely disappeared."
The "frozen salmon nasal cartilage" of Example 1 of Patent Document 1 corresponds to the "frozen salmon nasal cartilage blocks" described above. The term "dry basis" means a dry mass basis.

### Analysis of Proteoglycan Extraction Using Small Pieces of Frozen Salmon Nasal Cartilage

Extraction of proteoglycan was attempted by adding water to small pieces of frozen salmon nasal cartilage prepared as described above in item (2), and allowing the mixture to stand, or heating the mixture at 100°C. More specifically, analysis was carried out as follows. Four samples were prepared. Each sample was prepared by adding 60 mL of distilled water to about 12 g of the small pieces of frozen salmon nasal cartilage. These four samples were heated at 100°C for 1 hour, 2 hours, 3 hours, and 4 hours, respectively, and each sample was centrifuged with a centrifugal separator at 5,000 rpm at 4°C for 20 minutes to remove insoluble matter (residue) and collect the supernatant. After the amount of the collected supernatant was measured, the amount of uronic acid in the supernatant was measured by the carbazole-sulfuric acid method. (The amount of uronic acid reflects the amount of proteoglycan. In the tables and figures, the amount of uronic acid may be represented using the term "GlcA," an abbreviation of glucuronic acid, which is a uronic acid.) In addition, the amount of protein in the collected supernatant was measured by the Bradford method. The results are shown in Table 1. Fig. 2 is a graphical representation of Table 1.

**Table 1**

| Heating time (amount of small pieces (g)) | Amount of liquid (mL) | Per gram of small pieces of frozen salmon nasal cartilage | | GlcA/protein | Residue amount (g) and state |
|---|---|---|---|---|---|
| | | GlcA (mg) | Protein (mg) | | |
| 1 hour (12.261 g) | 31.0 | 4.507 | 0.441 | 10.22 | 33.83 Slightly soft |
| 2 hours (12.322 g) | 40.0 | 8.069 | 0.883 | 9.14 | 28.49 Jelly-like state; shape easily collapses when pressed with a finger |
| 3 hours (12.599 g) | 45.5 | 8.636 | 1.039 | 8.31 | 21.79 About to lose its shape |
| 4 hours (12.405 g) | 61.5 | 15.592 | 3.401 | 4.58 | 5.56 No shape, mushy; small amount |

From Table 1 and Fig. 2, it was found that the extraction amount of proteoglycan increases as the heating time is increased. It was also found that heating for more than 3 hours (e.g., 4 hours), in particular, increases the extraction amount of proteoglycan significantly.

### Comparison of Small Pieces of Frozen Salmon Nasal Cartilage, Frozen Salmon Nasal Cartilage Blocks, and Defatted Salmon Nasal Cartilage Powder

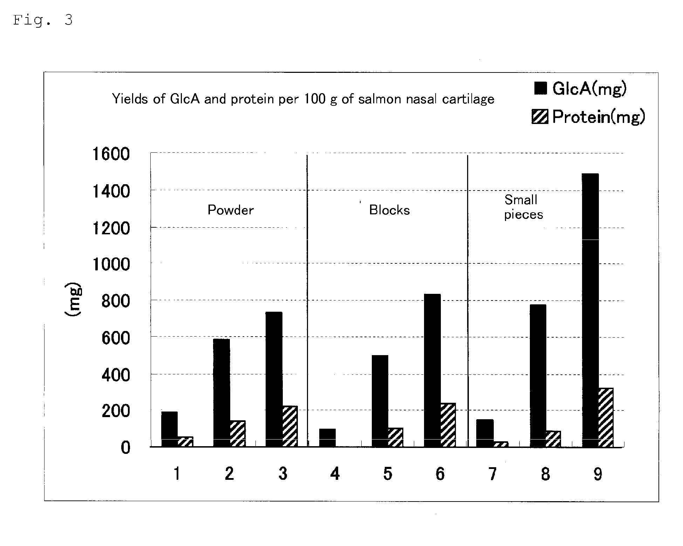

Proteoglycan was also extracted from the frozen salmon nasal cartilage blocks from item (1) and from the defatted salmon nasal cartilage powder from item (3) in the same manner as for the small pieces of frozen salmon nasal cartilage from item (2) above (except that the heating time and the heating temperature were changed), and the extraction amounts thereof were compared. The results are shown in Table 2. In Table 2, the amount of extracted uronic acid and the amount of extracted protein are represented in terms of amounts per 100 g of the frozen salmon nasal cartilage blocks. The amount of protein was measured by the Bradford method. Fig. 3 is a graphical representation of Table 2. The descriptions of the samples (1 to 9) shown in Fig. 3 correspond to the descriptions of 1 to 9 of the Sample No. column in Table 2.

**Table 2**

| Extraction amounts per 100 g of the Frozen Salmon Nasal Cartilage Blocks (defatted salmon nasal cartilage powder: 5.83 g, small pieces of frozen salmon nasal cartilage: 95.6 g) | | | | | | |
|---|---|---|---|---|---|---|
| | Sample No. | Heating temperature | Heating time | GlcA (mg) | Protein (mg) | GlcA/protein |
| Defatted salmon nasal cartilage powder | 1 | 4°C | 2 hours | 186.68 | 45.88 | 4.07 |
| | 2 | 100°C | 2 hours | 581.43 | 136.25 | 4.27 |
| | 3 | 100°C | 4 hours | 733.15 | 215.30 | 3.41 |
| Frozen salmon nasal cartilage blocks | 4 | 4°C | 2 hours | 96.90 | - | - |
| | 5 | 100°C | 2 hours | 499.90 | 96.90 | 5.16 |
| | 6 | 100°C | 4 hours | 832.10 | 234.85 | 3.54 |
| Small pieces of frozen salmon nasal cartilage | 7 | 4°C | 2 hours | 150.67 | 18.55 | 8.12 |
| | 8 | 100°C | 2 hours | 771.40 | 84.41 | 9.14 |
| | 9 | 100°C | 4 hours | 1490.60 | 325.14 | 4.58 |

When the frozen salmon nasal cartilage blocks were simply subjected to water extraction and were not heated, protein was not extracted. It was thus found that proteoglycan is not extracted under such extraction conditions.

It was found that the amount of extracted uronic acid (which reflects the amount of proteoglycan) significantly increases, in particular, when the small pieces of frozen salmon nasal cartilage are heated in water at 100°C for 4 hours.

### Analysis of Molecular Weight of Proteoglycan

To 20 g of the defatted salmon nasal cartilage powder described above, 1,000 mL of purified water at room temperature (pH of 6.5) was added, and the mixture was stirred for 30 minutes and then centrifuged (8,000 rpm, 30 minutes, 4°C) to collect the supernatant. The supernatant was freeze-dried to obtain a water extract of fish cartilage. The water extract of fish cartilage may be hereinafter referred to as Sample No. 10.

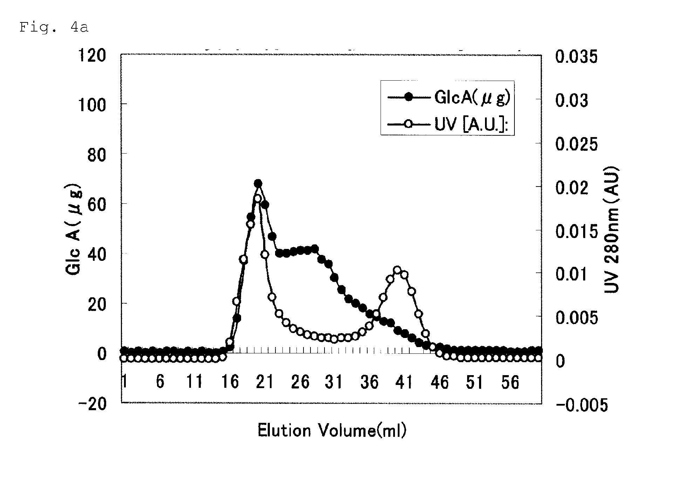

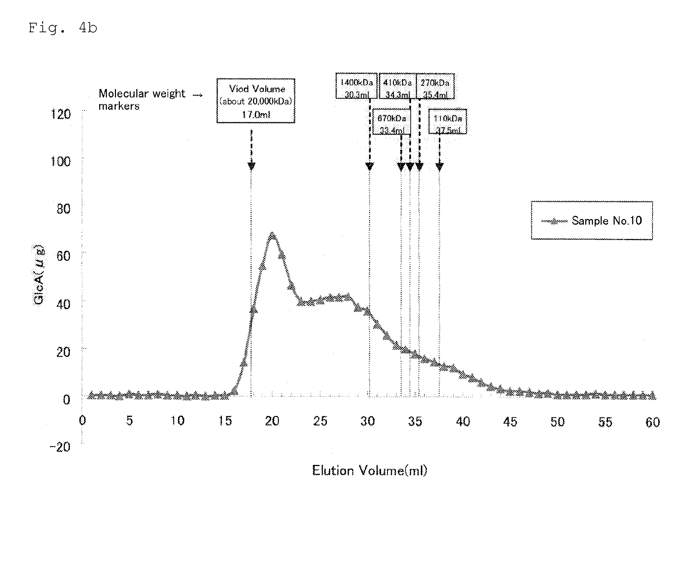

Sample No. 10 was separated into fractions by gel filtration chromatography under the conditions described below. The amount of uronic acid contained in each fraction was quantified by the carbazole-sulfuric acid method. In addition, absorbency at 280 nm of each fraction was measured, and the absorbency was defined as a value reflecting the amount of protein contained therein. Based on these results, a uronic acid amount chromatogram and a 280-nm protein amount chromatogram were drawn. Fig. 4a shows a superimposition of the uronic acid amount chromatogram and the 280-nm protein amount chromatogram. Fig. 4b shows the uronic acid amount chromatogram together with the position of each fraction at which each molecular weight marker was eluted. Since the amount of each fraction in the gel filtration chromatography was 1 mL/tube as described below, the horizontal axis, "Elution Volume (mL)", in Fig. 4 also reflects the fraction No.

### Gel Filtration Chromatography Conditions

Column: Sepharose CL-2B packed column (1-cm dia. × 50 cm column packed with Sepharose CL-2B as a carrier. Sepharose CL-2B has a dextran fractionation range of 100 to 20,000 kDa and is available from GE Healthcare and other companies. Sepharose CL-2B, CAS registry No. 65099-79-8, is a 2% crosslinked agarose with a particle size of 60 to 200 µm (measured by the laser diffraction scattering method).)
Buffer: 0.1 M phosphate buffer (pH of 7.1, containing 0.2 M NaCl) Amount of applied sample: 4 mg of Sample No. 10 (dissolved in 1 mL of buffer for use)
Flow rate: 0.15 mL/min
Amount of fraction: 1 mL/tube
Molecular weight analytical curve: The various dextran molecular weight markers described below were subjected to gel filtration chromatography under the same conditions as described above (except that the amount of the sample was 1 mg/1 mL of buffer), and absorbency (which reflects the amount of dextran) of each fraction was measured by the phenol-sulfuric acid method, and an analytical curve was prepared.

### Dextran Molecular Weight Markers

Dextran from *Leuconostoc mesenteroides* (mol wt 5,000,000 - 40,000,000)(Sigma) ... For measuring the void volume of the column, 20,000 kDa
Dextran Standard 1,400,000 (Sigma) ... 1400 kDa
Dextran Standard 670,000 (Sigma) ... 670 kDa
Dextran Standard 410,000 (Sigma) ... 410 kDa
Dextran Standard 270,000 (Sigma) ... 270 kDa

The dextran from *Leuconostoc mesenteroides* was used after pretreatment was carried out to remove low molecular weight dextran contained in the marker. The pretreatment was performed by eluting dextran from *Leuconostoc mesenteroides* under the conditions described above in "Gel Filtration Chromatography Conditions" (the applied amount was the amount for marker) and collecting and freeze-drying fractions having a molecular weight of not less than 20,000 kDa. More specifically, fractions that corresponded to a peak that appeared first in a chromatogram reflecting the amount of dextran were collected and freeze-dried. The chromatogram was prepared by measuring the absorbency of each fraction by the phenol-sulfuric acid method. (It is believed that dextran having a molecular weight of not less than 20,000 kDa was obtained by collecting and freeze-drying such fractions.) This freeze-dried product was actually used as a marker (for measuring the void volume of the column).
Measurement of absorbency to obtain a chromatogram reflecting the amount of dextran was performed according to the method (the phenol-sulfuric acid method) described in Hodge, J. E. and Hofreiter, B. T., Method in Carbohydrate Chemistry, 1, 338 (1962). More specifically, the measurement was carried out as follows.
[1] 500 µL of a sample aqueous solution is placed in a 105 × 15 mm test tube.
[2] 500 µL of a phenol reagent (5 v/v% aqueous phenol solution) is added thereto, and the mixture is stirred.
[3] 2.5 mL of concentrated sulfuric acid is added thereto, and immediately the mixture is stirred vigorously for 10 seconds.
[4] The mixture is left to stand for 20 minutes or more at room temperature.
[5] The absorbency at 490 nm is measured with a spectrophotometer. The obtained analytical curve was (y = -4.1355Ln(x) + 59.47; R² = 0.9869). From the R² value, it was found that the molecular weight and the fraction No. (i.e., elution volume) were highly correlated.

As shown in Fig. 4b, Sample No. 10 was found to contain proteoglycan having a molecular weight of not less than 2,000,000 (200 × 10⁴) (further, a molecular weight of not less than 2,500,000 (250 × 10⁴)). As shown in Fig. 4b, the ratio of the component having not less than (or not greater than) a specific molecular weight contained in the water extract of fish cartilage can be determined by drawing a vertical line from an elution volume point corresponding to the specific molecular weight in the uronic acid amount chromatogram and calculating the ratio of the two areas of the chromatogram divided by the vertical line.

Next, Sample No. 10 was analyzed in more detail. More specifically, the analysis was carried out as follows. Sample No. 10 was subjected to ion-exchange chromatography under the conditions described below to fractionate proteoglycan. More specifically, 16 mL eluted fractions were collected to quantify the amount of uronic acid by the carbazole-sulfuric acid method.

### Ion-Exchange Chromatography Conditions

Column: DEAE packed column (5.0-cm dia. × 20 cm column packed with DEAE (GE Healthcare) as a carrier)
Buffer: 7 M urea-Tris-hydrochloric acid buffer (pH of 7.2) Gradient: NaCl concentration 0 → 0.75 M
Amount of applied sample: 200 mg of Sample No. 10 (freeze-dried product of a water extract of fish cartilage) (dissolved in 50 mL of buffer and applied)
Flow rate: 2.0 mL/min
Amount of fraction: 16 mL/tube

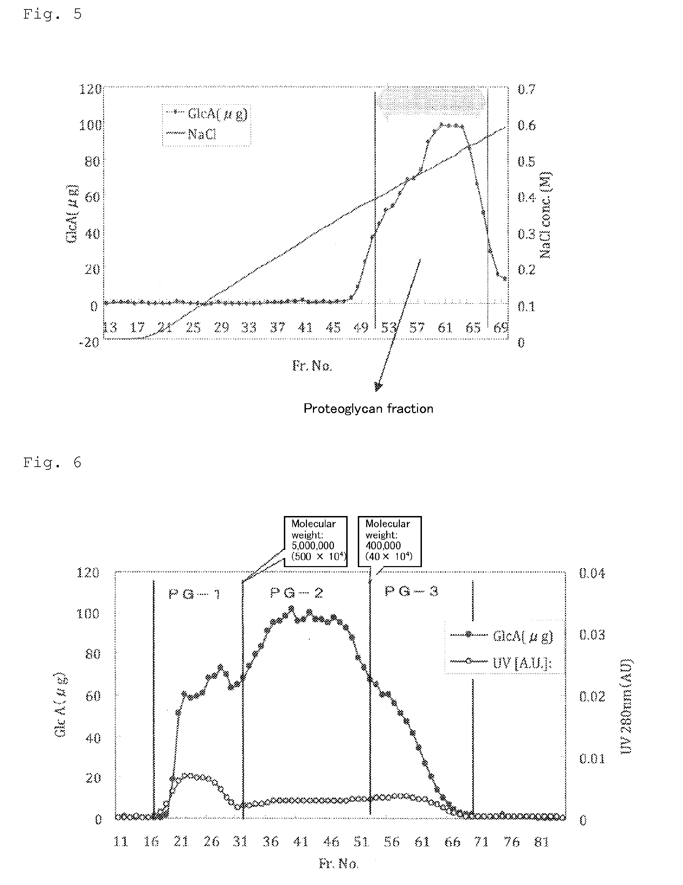

The obtained uronic acid amount chromatogram was shown in Fig. 5. Fractions containing uronic acid (shown in Fig. 6 as a left-right arrow) were collected, dialyzed, and then freeze-dried to obtain powder. The powder was used as a "proteoglycan fraction" for the following analysis.

The proteoglycan fraction obtained as described above was subjected to gel filtration chromatography under the following conditions and fractionated. The amount of uronic acid contained in each fraction was quantified by the carbazole-sulfuric acid method. In addition, absorbency at 280 nm of each fraction was measured and the absorbency was defined as a value reflecting the amount of protein contained therein. Based on these results, a uronic acid amount chromatogram and a 280-nm protein amount chromatogram were drawn.

### Gel Filtration Chromatography Conditions

Column: Sepharose CL-2B packed column (5.0-cm dia. × 50 cm column packed with Sepharose CL-2B (GE Healthcare) as a carrier)
Buffer: 0.1 M phosphate buffer (pH of 7.1, containing 0.2 M NaCl) Amount of applied sample: 150 mg
Flow rate: 2.0 mL/min
Amount of fraction: 16 mL/tube

The various dextran molecular weight markers described below were also subjected to gel filtration chromatography under the same conditions (except that the amount of the sample was 50 mg), and absorbency of each eluted fraction (which reflects the amount of dextran) was measured by the phenol-sulfuric acid method. An analytical curve of molecular weights and fraction Nos. was obtained, and fraction Nos. having a molecular weight of 5,000,000 (500 × 10⁴) and a molecular weight of 400,000 (40 × 10⁴) were determined.

### Dextran Molecular Weight Markers

Dextran from *Leuconostoc mesenteroides* (mol wt 5,000,000 - 40,000,000)(Sigma) ... 20,000 kDa
Dextran Standard 1,400,000 (Sigma) ... 1,400 kDa
Dextran Standard 270,000 (Sigma) ... 270 kDa

The dextran from *Leuconostoc mesenteroides* was used after pretreatment was carried out to remove low molecular weight dextran contained in the marker. The pretreatment was performed by eluting dextran from *Leuconostoc mesenteroides* under the conditions described above in "Gel Filtration Chromatography Conditions" (the applied amount was the amount in the marker treatment), and collecting and freeze-drying fractions having a molecular weight of not less than 20,000 kDa. More specifically, fractions that corresponded to a peak that first appeared in a chromatogram reflecting the amount of dextran were collected and freeze-dried. (It is believed that dextran having a molecular weight of not less than 20,000 kDa was obtained by collecting and freeze-drying such fractions.) This freeze-dried product was actually used as a marker (for measuring the void volume of the column). Measurement of absorbency to obtain a chromatogram reflecting the amount of dextran was performed in the same manner as described above.

The obtained chromatograms were shown in Fig. 6. Fractions containing uronic acid were divided into three groups: a group having a molecular weight of not less than 5,000,000 (500 × 10⁴), a group having a molecular weight of not less than 400,000 (40 × 10⁴) to less than 5,000,000 (500 × 10⁴), and a group having a molecular weight of less than 400,000 (40 × 10⁴). The fractions were collected, dialyzed, and then freeze-dried to obtain three types of water extracts (powder) of fish cartilage having different molecular weights. These three types of water extracts of fish cartilage were used as PG-1 extract (containing proteoglycan having a molecular weight of not less than 5,000,000 (500 × 10⁴)), PG-2 extract (containing proteoglycan having a molecular weight of not less than 400,000 (40 × 10⁴) to less than 5,000,000 (500 × 10⁴)), and PG-3 extract (containing proteoglycan having a molecular weight of less than 400,000 (40 × 10⁴)). From the results, it was confirmed that Sample No. 10 contained proteoglycan having a molecular weight of not less than 5,000,000 (500 × 10⁴).

### Evaluation of Skin Anti-aging Ability through Oral Administration (1)

A water extract of fish cartilage was actually given to mammals, and the effects of the extract were analyzed.

### Experimental Animal Used

Hairless mice (Hr-/Kud, male) (Kyudo Co., Ltd.) were used for the experiment. Male mice (6 weeks old), which are free of the influence of fluctuations in estrogen on skin conditions, were preliminarily fed, and then used for the experiment.

### Experimental Method

The mice were placed in three feeding cages as shown in Table 3 to be divided into groups (Groups 1 to 3)(six mice in one group). In addition, the subjects were marked on their tail portion to be individually identified. They continued to be preliminarily fed until the start of analysis. The average body weight during the experiment was 37 g. Sample No. 10 was used as the water extract of fish cartilage for Group 3 in Table 3, which is an evaluation material.

**Table 3**

| Group | Evaluation material | UVB irradiation |
|---|---|---|
| 1 | Water (control) | No |
| 2 | Water (control) | Yes |
| 3 | Water extract of fish cartilage (0.175 mg/day/mouse) | Yes |

### Preparation of Oral Administration Sample of Evaluation Material

Sample No. 10 was dissolved in water to 0.35 mg/mL to prepare an administration sample.

### Oral Administration Method

When the hairless mice reached 8 weeks of age, 0.5 mL of the administration sample was given once a day through forced oral administration using a sonde (0.175 mg/day/mouse). To the controls, 0.5 mL of distilled water was given. This administration was continued at a frequency of 6 times per week (once a day for 6 days, Monday to Saturday) until the end of the experiment.

### UVB Irradiation Method

UVB irradiation started 3 weeks after oral administration began. The mice were placed in a cage for UVB irradiation. The cage was placed into a UVB irradiation device to carry out UVB irradiation 6 times per week (once a day for 6 days, Monday to Saturday), at an intensity of 1.0 mW/cm². The amount of irradiation was 60 mJ/cm² during only the first week after the start of the irradiation, and 120 mJ/cm² from the second week onward. Note that UVB was ultraviolet with a wavelength from 280 to 315 nm.

### Evaluation of Anti-inflammation

Erythema values after 4 weeks and 7 weeks of UVB irradiation were measured with a Mexameter, a multiprobe skin measuring instrument (MPA580: Courage-Khazaka), to evaluate anti-inflammation. Five areas on the back of each mouse were measured, and the average value was calculated. Inflammation due to UVB irradiation causes erythema. Thus, a larger erythema value means severer inflammation.

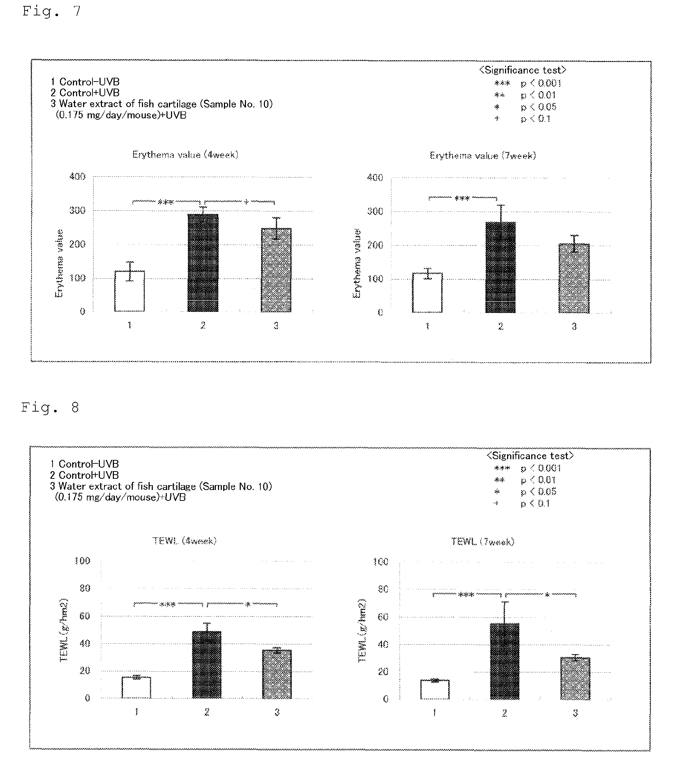

Fig. 7 shows the results of measuring erythema values after 4 weeks and 7 weeks of UVB irradiation. From the results, it was found that erythema is reduced through oral administration of Sample No. 10, which is a water extract of fish cartilage. This indicates that the water extract of fish cartilage reduces inflammation caused by ultraviolet irradiation.

### Evaluation of Skin Barrier Functionality

Transepidermal water loss (TEWL) after 4 weeks and 7 weeks of UVB irradiation was measured with a Tewameter, a multiprobe skin measuring instrument (MPA580: Courage-Khazaka), to evaluate skin barrier functionality. Three areas on the back of each mouse were measured, and the average value was calculated. A larger TEWL value means lower skin barrier functionality (functions that prevent foreign matter outside the skin from penetrating into the body, and that prevent moisture inside the body from escaping to the outside).
Fig. 8 shows the results of measuring TEWL after 4 weeks and 7 weeks of UVB irradiation. The results reveal that oral administration of Sample No. 10, which is a water extract of fish cartilage, reduces the TEWL value and improves skin barrier functionality.

### Evaluation of Skin Anti-aging Ability through Oral Administration (2) (Analysis of Difference in Effect due to Difference in Molecular Weight)

Skin anti-aging ability was analyzed using Sample No. 10, PG-1 extract, and PG-2 extract. PG-1 extract and PG-2 extract were obtained by further purifying Sample No. 10.

### Experimental Animal Used

Hairless mice (Hr-/Kud, male) (Kyudo Co., Ltd.) were used for the experiment. Male mice (6 weeks old), which are free of the influence of fluctuations in estrogen on skin conditions, were preliminarily fed, and then used for the experiment.

### Experimental Method

The mice were placed in five feeding cages, as shown in Table 4, to be divided into groups (Groups 1 to 5)(six mice in one group). In addition, the subjects were marked on their tail portion to be individually identified. They continued to be preliminarily fed until the start of analysis.

**Table 4**

| Group | Evaluation material | UVB irradiation |
|---|---|---|
| 1 | Water (control) | No |
| 2 | Water (control) | Yes |
| 3 | Sample No. 10 | Yes |
| 4 | PG-1 extract | Yes |
| 5 | PG-2 extract | Yes |

### Preparation of Oral Administration Samples of Evaluation

### Materials

Evaluation materials were individually dissolved in water to 0.2 mg/mL to prepare administration samples.

### Oral Administration Method

When the hairless mice reached 8 weeks of age, 0.5 mL of each administration sample was individually given once a day through forced oral administration using a sonde (0.1 mg/day). To the controls, 0.5 mL of distilled water was given. This administration was continued at a frequency of 6 times per week (once a day for 6 days, Monday to Saturday) until the end of the experiment.

### UVB Irradiation Method

UVB irradiation started 3 weeks after the start of the oral administration. The mice were placed in a cage for UVB irradiation. The cage was placed into a UVB irradiation device to carry out UVB irradiation 6 times per week (once a day for 6 days, Monday to Saturday), at an intensity of 1.0 mW/cm². The amount of irradiation was 60 mJ/cm² during only the first week after the start of the irradiation, and 120 mJ/cm² from the second week onward. Note that UVB was ultraviolet with a wavelength from 280 to 315 nm.

### Evaluation of Anti-inflammation

Erythema values after 4 weeks and 7 weeks of UVB irradiation were measured with a Mexameter, a multiprobe skin measuring instrument (MPA580: Courage-Khazaka), to evaluate anti-inflammation. Five areas on the back of each mouse were measured, and the average value was calculated. Inflammation due to UVB irradiation causes erythema. Thus, a larger erythema value means severer inflammation.

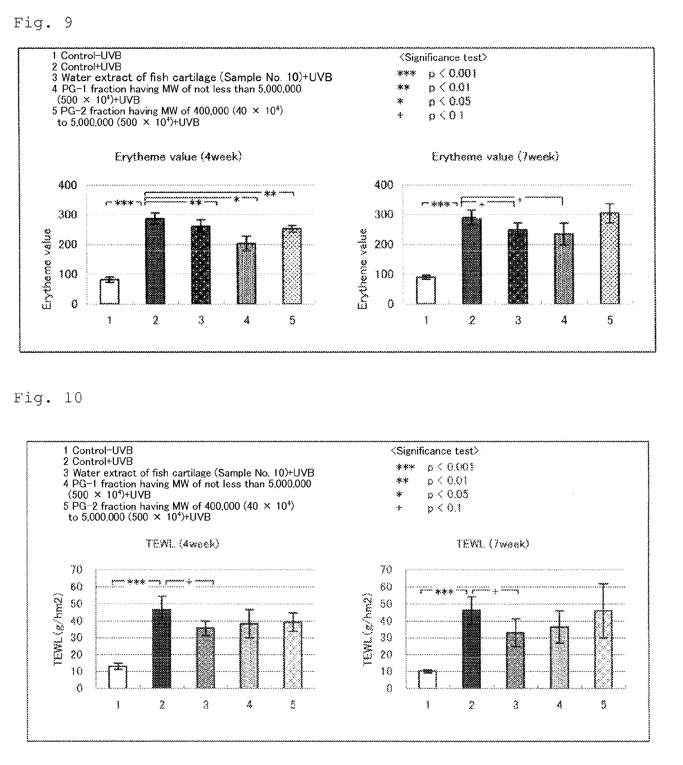

Fig. 9 shows the results of measuring erythema values after 4 weeks and 7 weeks of UVB irradiation. From the results, it was found that oral administration of the water extract of fish cartilage used reduces erythema, and thus reduces inflammation caused by ultraviolet irradiation. It was also found that in particular, high molecular weight proteoglycan (PG-1 extract) having a molecular weight of not less than 5,000,000 (500 × 10⁴) exhibits a large effect among the proteoglycans contained in the water extract of fish cartilage.

### Evaluation of Skin Barrier Functionality

Transepidermal water loss (TEWL) after 4 weeks and 7 weeks of UVB irradiation was measured with a Tewameter, a multiprobe skin measuring instrument (MPA580: Courage-Khazaka), to evaluate skin barrier functionality. Three areas on the back of each mouse were measured, and the average value was calculated. A larger TEWL value means lower skin barrier functionality (functions that prevent foreign matter outside the skin from penetrating into the body, and that prevent moisture inside the body from escaping to the outside).
Fig. 10 shows the results of measuring TEWL after 4 weeks and 7 weeks of UVB irradiation. The results reveal that oral administration of the water extract of fish cartilage used reduces the TEWL value and improves skin barrier functionality.

### Evaluation of Skin Water Retention Ability

Stratum corneum water content after 4 weeks and 7 weeks of UVB irradiation was measured with a Corneometer, a multiprobe skin measuring instrument (MPA580: Courage-Khazaka), to evaluate skin water retention ability. Ten areas on the back of each mouse were measured, and the average value was calculated.

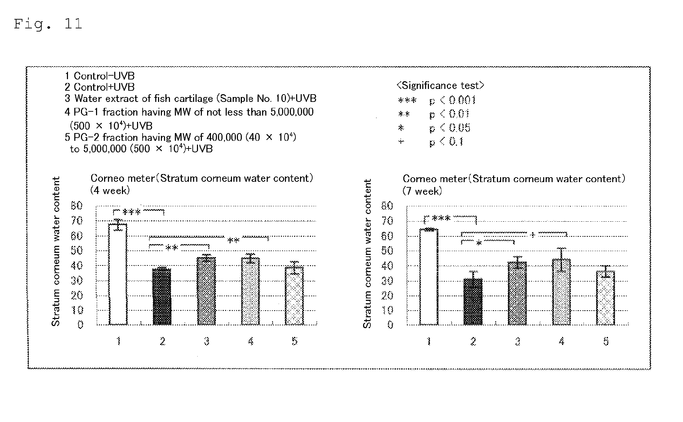

Fig. 11 shows the results of measuring TEWL after 4 weeks and 7 weeks of UVB irradiation. From the results, it was found that oral administration of the water extract of fish cartilage used increases stratum corneum water content and improves skin water retention ability. It was also found that, in particular, proteoglycan (PG-1 extract) having a molecular weight of not less than 5,000,000 (500 × 10⁴) exhibits a large effect among the proteoglycans contained in the water extract of fish cartilage.

The symbols in Figs. 7 to 11 mean the following.
*** : p < 0.001
** : p < 0.01
* : p < 0.05
+ : p < 0.1

### Evaluation of Skin Anti-aging Ability through Oral Administration (3) (Clinical Test)

The skin of the inner forearm and the upper inner arm of each subject (14 healthy females aged 25-55) was irritated with a chamber impregnated with sodium dodecyl sulfate (SLS) by the method described below, and changes after the irritation in the erythema disappearance rate (measured at the forearm) and in transepidermal water loss (TEWL) (measured at the upper arm) were compared. (The details of the test method are described below.)

Sample No. 10 (500 mg) used for the test group was inserted into six hard capsules, and crystalline cellulose powder (Ceolus (registered trademark) FD-301 (produced by Asahi Kasei Chemicals Corp.)) (500 mg) used as a placebo was inserted into six hard capsules. Each of them was orally administered to their respective subjects twice a day at a rate of three capsules each time (i.e., the daily intake was 500 mg). Sample No. 10 was orally administered to seven subjects, and the placebo was orally administered to seven subjects.

Sample No. 10 (500 mg) contained about 80 mg of proteoglycan having a molecular weight of not less than 5,000,000 (500 × 10⁴). This value (about 80 mg) was determined as follows. Fractions (Nos. 16 to 23) that correspond to proteoglycan having a molecular weight of not less than 5,000,000 (500 × 10⁴) in the uronic acid amount chromatogram of Sample No. 10 in Fig. 4 were collected, dialyzed, and freeze-dried to measure the dry weight thereof. The ratio of the dry weight to the amount (4 mg) of Sample No. 10 applied in the column for preparing the chromatogram in Fig. 4 was determined, and 500 mg was multiplied by this ratio to obtain the above value.

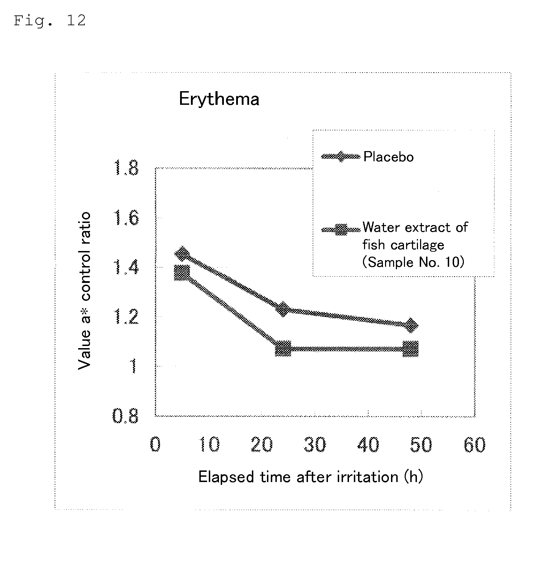

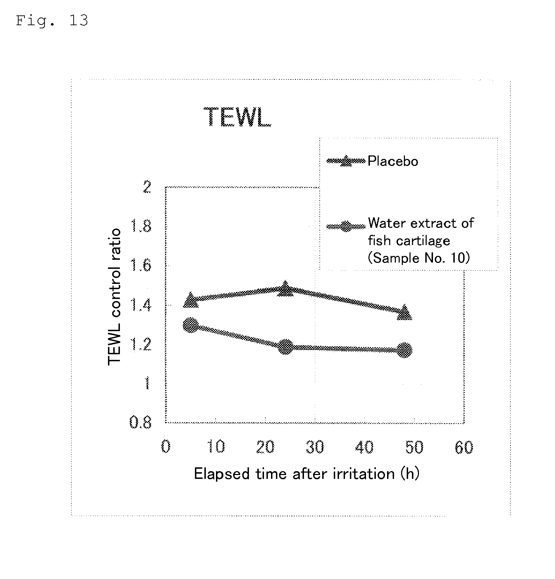

In the fifth week after the start of the administration (the administration continuing until the end of the measurement), a chamber impregnated with SLS was applied to the skin of the inner forearm and the upper inner arm of each subject to irritate the skin. Thereafter, the color of erythema on the forearm after the irritation (value a* in the L*a*b* color system) and the transepidermal water loss (TEWL) value of the upper arm after the irritation were measured at the irritated site and the non-irritated site of the arm. The relative value (control ratio) of the measured value of the irritated site to the measured value of the non-irritated site was calculated to examine changes in the erythema disappearance rate and in TEWL. The results are shown in Figs. 12 and 13.

More specifically, the skin irritation and the measurement were performed as follows.

### • Method of Irritation by SLS Patch Application and Measurement Method

After washing the inner arm, each subject was in a constant temperature, constant humidity room at 22°C with a relative humidity of 60% for 15 minutes or more. Subsequently, a chamber (200 large Finn Chambers on Scanpor, Norgeplaster Ltd.) with filter paper impregnated with 30 µL of 0.5% SLS (sodium dodecyl sulfate: Wako Pure Chemical Industries, Ltd., Lot. STQ8638) was applied to the skin on the inner arm of each subject to expose the skin to irritation under occlusion for 4 hours. Thereafter, the chamber was removed, and 1 hour, 1 day, and 2 days after the removal, the color of erythema at the irritated site (value a*) was measured with a CR-400 Chroma Meter (Konica Minolta), and the TEWL value at the irritated site was measured with a Tewameter TM-300 (Courage-Khazaka).

From Fig. 12, it was confirmed that oral administration of Sample No. 10 increases the rate at which erythema disappears (i.e., alleviates inflammation). From Fig. 13, it was also confirmed that oral administration of Sample No. 10 decreases the TEWL value (i.e., improves and enhances skin barrier functionality).

### Analysis of Cell Proliferation Effect

To 500 g of the small pieces of defatted salmon nasal cartilage described above, purified water was added in an amount that is 2.5 times the mass of the small pieces, and each mixture was heated at 100°C, 90°C, 70°C, or 50°C. The heating times at 100°C and 90°C were 3 hours each, and the heating times at 70°C and 50°C were 6 hours each. The cartilage was completely dissolved in the heating at 100°C and in the heating at 90°C, whereas the cartilage was not completely dissolved in the heating at 70°C or in the heating at 50°C, and thus the extraction ended with partially remaining cartilage. Centrifugation was performed for each mixture with a centrifugal separator at 8,000 rpm at 4°C for 30 minutes to remove insoluble matter (residue) and collect the supernatant, and the supernatant was freeze-dried to prepare freeze-dried samples (FD samples). These samples may be hereinafter referred to as a 100°C extraction FD sample, 90°C extraction FD sample, 70°C extraction FD sample, and 50°C extraction FD sample. In addition, a freeze-dried sample was obtained by heating a mixture of the small pieces of defatted salmon nasal cartilage and purified water at 100°C for 3 hours and then further for 3 hours and performing centrifugation and freeze drying in the same manner. This sample may be hereinafter referred to as a 100°C (3h) extraction FD sample.

Each of these FD samples was adjusted such that the GlcA concentration was 1 mg/1 mL of buffer and analyzed for the molecular weight of each sample by gel filtration chromatography in the same manner as the chromatography that was used to first analyze Sample No. 10 in the "Analysis of Molecular Weight of Proteoglycan" (the results of this chromatography being shown in Fig. 4) described above. More specifically, the percentage of the component having a molecular weight of not less than 5,000,000 (500 × 10⁴), the percentage of the component having a molecular weight of not less than 2,500,000 (250 × 10⁴), and the percentage of the component having a molecular weight of not less than 1,800,000 (180 × 10⁴) were determined from each uronic acid amount chromatogram. The results are shown in Table 5.

**Table 5**

| | ≥ 5,000,000 (500 × 10⁴) Da | ≥ 2,500,000 (250 × 10⁴) Da | ≥ 1,800,000 (180 × 10⁴) Da |
|---|---|---|---|
| 50°C extraction FD | 34.0% | 50.6% | 60.9% |
| 70°C extraction FD | 37.5% | 52.7% | 61.9% |
| 90°C extraction FD | 39.7% | 54.0% | 62.3% |
| 100°C extraction FD | 39.1% | 52.8% | 61.2% |
| 100°C (3 h) extraction FD | 26.2% | 41.1% | 51.1% |

Subsequently, the samples were analyzed for cell proliferation ability as follows. Specifically, in a culturing dish for each sample, human skin fibroblasts (HDF53: Cell Applications, Inc.) were seeded at 1.5 × 10⁴ cells in minimum essential medium (MEM) containing 10% fetal bovine serum (FBS). Each sample was filter-sterilized with a 22-µm filter and added individually to MEM to a concentration of 20 µg/mL. After the addition, the cells were cultured for five days. Following the culture, the MEM was removed, and the cells were detached and suspended with Trypsin-EDTA (Invitrogen). Thereafter, Trypan blue stain was added, and the number of cells was counted using a counting chamber.

Water was used as the control. Further, a product commercially available as proteoglycan (referred to as commercially available product A) was also analyzed in the same manner for comparison.

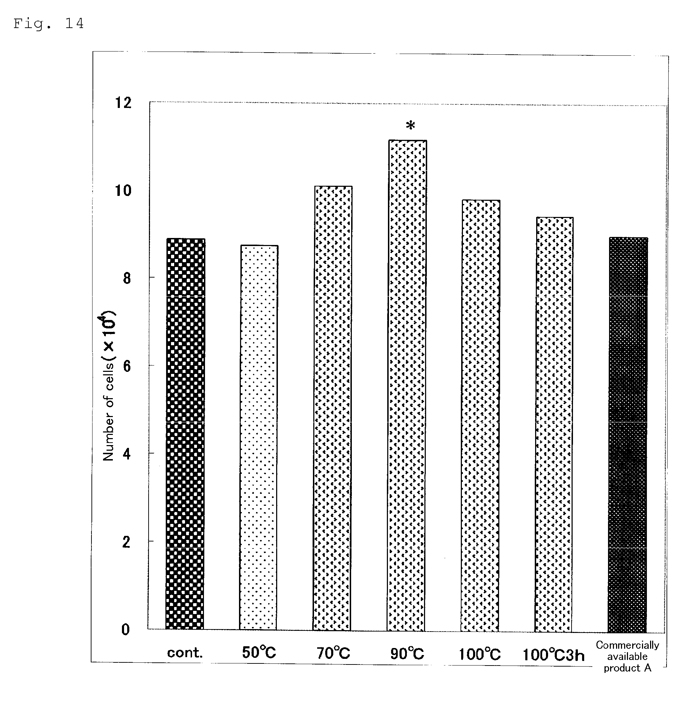

The results are shown in Fig. 14. The 90°C extraction FD sample significantly promoted cell proliferation compared to the control. The 70°C extraction FD sample and the 100°C extraction FD sample also showed a tendency to promote cell proliferation. In the analysis, the number of samples (n) was 11 for the control, 3 for the 50°C extraction FD sample, 6 for the 70°C extraction FD sample, 11 for the 90°C extraction FD sample, 6 for the 100°C extraction FD sample, 6 for the 100°C (3 h) extraction FD sample, and 5 for commercially available product A. Student's t-test was used for statistical processing.

Further, the composition of each sample used for the analysis was analyzed. Analysis of each component was performed as follows. For measurement of amounts of protein, lipid, ash, moisture, and hydroxyproline (used for measuring the amount of collagen), the samples were submitted to Japan Food Research Laboratories to have analysis conducted on components per 100 g of each sample. The amount of protein was measured by the Kjeldahl method, the amount of lipid was measured by the acid hydrolysis method, the amount of ash was measured by the direct ashing method, the amount of moisture was measured by the normal-pressure heat-drying method, and the amount of hydroxyproline was measured by automatic amino acid analysis (HPLC method). The amount of carbohydrate was calculated by subtracting the amounts of protein, lipid, ash, and moisture from 100 (g).

As described above, the amount of protein was measured by the Kjeldahl method, which uses the amount of nitrogen as an indicator. Since nitrogen derived from glycosaminoglycan is also measured, adjustment was carried out as follows.

To subtract nitrogen derived from glycosaminoglycan, which is not protein, the amount of the acidic saccharide component (in terms of chondroitin sulfate) was calculated from the amount of uronic acid determined by the carbazole-sulfuric acid method. (Specifically, the amount of uronic acid was multiplied by a conversion factor of 2.593). Since nitrogen derived from chondroitin sulfate corresponds to 2.9% of molecular weight, the obtained amount of the acidic saccharide component was multiplied by 2.9% to calculate nitrogen derived from glycosaminoglycan.

The nitrogen obtained by subtracting nitrogen derived from glycosaminoglycan includes nitrogen derived from collagen and nitrogen derived from protein other than collagen. Since the "protein factor" of collagen is different from that of the other protein, it is necessary to ascertain the amount of nitrogen derived from collagen and the amount of nitrogen derived from the other protein.

Collagen was quantified by quantifying "hydroxyproline," which is an amino acid specifically present in collagen, as described above. More specifically, collagen content was determined by measuring hydroxyproline (Hyp), which is an amino acid specifically present in collagen, and assuming that collagen contains 6.7% Hyp. The thus-obtained amount of collagen was divided by 5.55, which is the protein factor of collagen, to calculate the amount of nitrogen derived from collagen.

The amount of nitrogen derived from glycosaminoglycan and the amount of nitrogen derived from collagen were subtracted from the quantified value of nitrogen determined by the Kjeldahl method, and the value obtained by the subtraction was multiplied by 6.25, which is the protein factor of general protein, to calculate the amount of protein other than collagen.

The amount of protein was calculated by adding the amount of collagen to the amount of protein other than collagen determined as described above.

The results are shown in the following Table 6. The values in Table 6 represent grams per 100 g of extraction FD sample. Table 6 also shows the amount of proteoglycan having a molecular weight of not less than 1,800,000 (180 × 10⁴). The amount of proteoglycan having a molecular weight of not less than 1,800,000 (180 × 10⁴) was obtained by multiplying the amount of glycosaminoglycan listed in Table 6 by the percentage of the component having a molecular weight of not less than 1,800,000 (180 × 10⁴) listed in Table 5.

**Table 6**

| | | (1) 100°C extraction FD | (2) 90°C extraction FD | (2) 70°C extraction FD | (3) 50°C extraction FD | (4) 10% EtOH treatment → 90°C extraction FD | Commercially available product A (20% standard product) |
|---|---|---|---|---|---|---|---|
| Moisture | | 8.1 | 7.8 | 10.4 | 3.7 | 7.8 | 7.4 |
| Protein | | 54.6 | 52.9 | 35.2 | 36.5 | 56.9 | 7.1 |
| | Collagen in protein | 50.9 | 49.9 | 31.3 | 33.4 | 53.1 | 2.54 |
| Lipid | | 3.4 | 0.7 | 2.1 | 5 | 1.2 | 0.2 |
| Ash | | 8.2 | 7.7 | 9.7 | 12 | 7.2 | 5.3 |
| Carbohydrate | | 24.3 | 21.5 | 30.9 | 32.6 | 21.8 | 77.7 |
| Total | | 100 | 100 | 100 | 100 | 100 | 100 |
| Glycosaminoglycan | | 42.0 | 33.9 | 40.7 | 35.0 | 42.5 | 20 (standard value) |
| | Proteoglycan having a molecular weight of not less than 1,800,000 (180 × 10⁴) | 25.7 | 21.1 | 25.2 | 21.3 | 27.4 | ND |

| | | | | | | | |
|---|---|---|---|---|---|---|---|
| Unit: grams per 100 g of extraction FD sample Acidic saccharide: calculated as chondroitin sulfate equivalent amount Chondroitin sulfate equivalent amount = the amount of glucuronic acid × conversion factor of 2.593 | | | | | | | |

Further, the expression "10% EtOH treatment → 90°C extraction FD" in Table 6 refers to a sample obtained by defatting fish cartilage with 10% ethanol and then performing extraction with water at 90°C. More specifically, to 500 g of the small pieces of frozen salmon nasal cartilage, a 10% EtOH solution was added in an amount 2.5 times that of the small pieces, and the mixture was stirred with a stirrer at room temperature for 24 hours and centrifuged at 8,000 rpm at 4°C for 30 minutes. The collected insoluble matter (residue containing cartilage) was heated until the cartilage was completely dissolved through extraction at 90°C with propeller stirring, and then centrifugation and filtration were performed in the same manner as in the FD samples shown in Table 5 to obtain the freeze-dried (FD) sample.

As shown in Table 6, most of the protein contained in the water extract of fish cartilage according to the present invention is collagen. Further, in particular, it can be seen that the smaller the lipid content or ash content is, the more the water extract of fish cartilage has cell proliferation ability (i.e., exhibits the cell proliferation promoting effect). It is believed that the water extract of fish cartilage having cell proliferation ability more efficiently provides the inflammation alleviation effect or anti-aging effect.

## Claims

1. A water extract of fish cartilage comprising a proteoglycan having a molecular weight of not less than 1,800,000 (180×10⁴).

2. The water extract of fish cartilage according to claim 1, comprising a proteoglycan having a molecular weight of not less than 2,500,000 (250×10⁴).

3. The water extract of fish cartilage according to claim 2, comprising a proteoglycan having a molecular weight of not less than 5,000,000 (500×10⁴).

4. The water extract of fish cartilage according to claim 3, comprising, on a dry mass basis, 9 mass% or more of a proteoglycan having a molecular weight of not less than 5,000,000 (500×10⁴).

5. The water extract of fish cartilage according to any one of claims 1 to 4, comprising, on a dry mass basis, 30 mass% or more of collagen.

6. The water extract of fish cartilage according to any one of claims 1 to 5, wherein the fish is salmon or trout, and the cartilage is nasal cartilage.

7. A food/drink composition, an oral composition, or a cosmetic composition comprising the water extract of fish cartilage according to any one of claims 1 to 6.

8. The composition according to claim 7, for use in alleviation of inflammation or anti-aging.
